# EUROPEAN PATENT SPECIFICATION

(11) **EP 4 051 190 B1**
(45) Date of publication and mention of the grant of the patent: **22.05.2024**
(21) Application number: 20804411.5
(22) Date of filing: 27.10.2020
(51) Int. Cl.: A61F 5/44, A61B 10/00, A61F 5/453, A61F 5/455, A61F 5/451

(54) **FLUID COLLECTION ASSEMBLIES INCLUDING A SAMPLE PORT**
FLUIDSAMMELANORDNUNGEN MIT EINEM PROBENPORT
ENSEMBLES DE COLLECTE DE FLUIDE COMPRENANT UN ORIFICE D'ÉCHANTILLON

(30) Priority: 28.10.2019 US 201962926767 P
(43) Date of publication of application: 07.09.2022
(73) Proprietor: Purewick Corporation, El Cajon, California 92020 (US)
(72) Inventor: BROOKS, Christopher K., Decatur, Georgia 30033 (US)
(74) Representative: Hoffmann Eitle
(86) International application number: PCT/US2020/057562
(87) International publication number: WO 2021/086868

(56) References cited:
- EP-A1- 1 616 542
- WO-A1-2021/025919
- PT-E- 2 068 717
- US-A1- 2013 245 496
- US-A1- 2015 290 425
- US-A1- 2017 266 031
- US-A1- 2017 325 788
- US-A1- 2018 228 642

## Description

### BACKGROUND

A person or animal may have limited or impaired mobility such that typical urination processes are challenging or impossible. For example, a person may experience or have a disability that impairs mobility. A person may have restricted travel conditions such as those experienced by pilots, drivers, and workers in hazardous areas. Additionally, sometimes urine collection is needed for monitoring purposes or clinical testing.

Urinary catheters, such as a Foley catheter, can be used to address some of these circumstances, such as incontinence. Unfortunately, urinary catheters can be uncomfortable, painful, and can lead to complications, such as infections. Further, obtaining samples from urinary catheters, under certain circumstances, may be challenging or difficult under certain circumstances.

US 2017/325788 A1 discloses a device for collecting a sample of urine discharged from the urethra of a female. It includes a conduit for passage of urine surplus to that needed for the sample.

WO 2021/025919 A1 is an earlier patent application of the Applicant, published later than the PCT filing date of the present application. US2018/0228642 A1 and US2017/266031 A1 also disclose similar devices for collecting urine discharged from a body of a user. US 2018/228642 A1 discloses a device for collecting urine discharged from a body of a user includes a fluid collection assembly having at least one layer for drawing urine discharged from the body into an interior cavity, an external covering that covers a portion of the at least one layer, and at least one fenestration for receiving urine, wherein the fenestration is a portion of the fluid collection assembly that is uncovered by the external covering. The device further includes a cap enclosing a first end of the assembly, a tube having a first end in fluid communication with the cap, and a shape retaining element configured to conform the assembly to a curved configuration and maintain the curved configuration until the configuration is adjusted. The assembly is configured to be disposed against the body of the user, with the at least one fenestration in operative relation with a urethral opening of the user.

### SUMMARY OF THE INVENTION

The invention is as defined by the claims. A fluid collection assembly according to the invention is defined in claim 1. A further aspect of the invention is given by a method of using the fluid collection assembly in a fluid collection system defined by claim 14. The dependent claims are directed to optional features and preferred embodiments.

### OUTLINE OF THE DISCLOSURE

Below, a fluid collection assembly is disclosed. The fluid collection assembly includes a fluid impermeable barrier including a first end region and a second end region longitudinally spaced from the first end. The fluid impermeable barrier includes a sump that forms at least a portion of the first end region. The fluid impermeable barrier defines at least one opening, a chamber in fluid communication with the at least one opening, a fluid reservoir defined by at least a portion of the sump, and a fluid outlet in fluid communication with the fluid reservoir. The fluid collection assembly also includes at least one wicking material disposed in the fluid impermeable barrier. Further, the fluid collection assembly includes a sample port attached to the sump and in fluid communication with the fluid reservoir. The sample port is configured to switch between a closed state that restricts fluid flow through the sample port and an open state that permits fluid flow through the sample port. The sample port is configured to be connected to a fluid collector.

In addition, a fluid collection system is disclosed. The fluid collection system includes a fluid collection assembly and a fluid collector. The fluid collection assembly includes a fluid impermeable barrier including a first end region and a second end region longitudinally spaced from the first end. The fluid impermeable barrier includes a sump that forms at least a portion of the first end region. The fluid impermeable barrier defines at least one opening, a chamber in fluid communication with the at least one opening, a fluid reservoir defined by at least a portion of the sump, and a fluid outlet in fluid communication with the fluid reservoir. The fluid collection assembly also includes at least one wicking material disposed in the fluid impermeable barrier. Further, the fluid collection assembly includes a sample port attached to the sump and in fluid communication with the fluid reservoir. The sample port is configured to switch between a closed state that restricts fluid flow through the sample port and an open state that permits fluid flow through the sample port. The sample port is configured to be connected to a fluid collector.

Also, a method of using a fluid collection system is disclosed. The method includes attaching the fluid collector to the sample port. The method also includes receiving bodily fluids from an individual through the at least one opening, into the chamber, and to the fluid reservoir. The method further includes removing at least some of the bodily fluids that are present in the fluid reservoir with the fluid collector. Additionally, the method includes detaching the fluid collector from the sample port. Further, the method includes switching the sample port from the open state to the closed state. The method additionally includes removing substantially all of a remainder of the bodily fluids present in the fluid reservoir through the fluid outlet using a suction force.

Features from any of the disclosed embodiments of the invention may be used in combination with one another, without limitation. In addition, other features and advantages of the present invention will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1** **A** is a schematic isometric view of a fluid collection assembly.
**FIG. 1B** is a cross-sectional view of the fluid collection assembly.
**FIGS. 1C** and **1D** are isometric views of a portion of different fluid collection systems.
FIG. 2 is excised.
**FIG. 3A** is a schematic cross-sectional view of a fluid collection assembly.
**FIG. 3B** is a schematic cross-sectional view of a fluid collection assembly having an elongated fluid reservoir.
**FIGS. 4A** to **4C** are schematic cross-sectional views of fluid collection assemblies including sumps exhibiting a greater rigidity than the rest of the fluid impermeable barriers thereof.
FIG. 5 is excised.
**FIG. 6** is a schematic illustration of fluid collection system that includes a fluid collection assembly.

### DETAILED DESCRIPTION

Fluid collection assemblies including a sample port, systems including the same, and methods of using the same are disclosed below. An example fluid collection assembly includes a fluid impermeable barrier including a first end region, a second end region opposite the first end region, and a sump that forms at least a portion of the first end region. The fluid impermeable barrier also defines at least one opening between the first end region and the second end region and a fluid reservoir that is defined by at least a portion of the sump. The fluid collection assembly also includes at least one wicking material disposed in the chamber and a sample port extending from the sump. The sample port is configured to be connected to a fluid collector (e.g., a syringe or a vial). The sample port is in fluid communication with the fluid reservoir such that a sample of the bodily fluids (e.g., urine) received by the fluid collection assembly may be obtained.

During use, the opening of the fluid impermeable barrier is positioned adjacent to the urethral opening of an individual. Bodily fluids (e.g., urine) that are emitted from the urethral opening are received by the wicking material through the opening and flow through the wicking material to the fluid reservoir. The bodily fluids are removed from the fluid reservoir using a suction force.

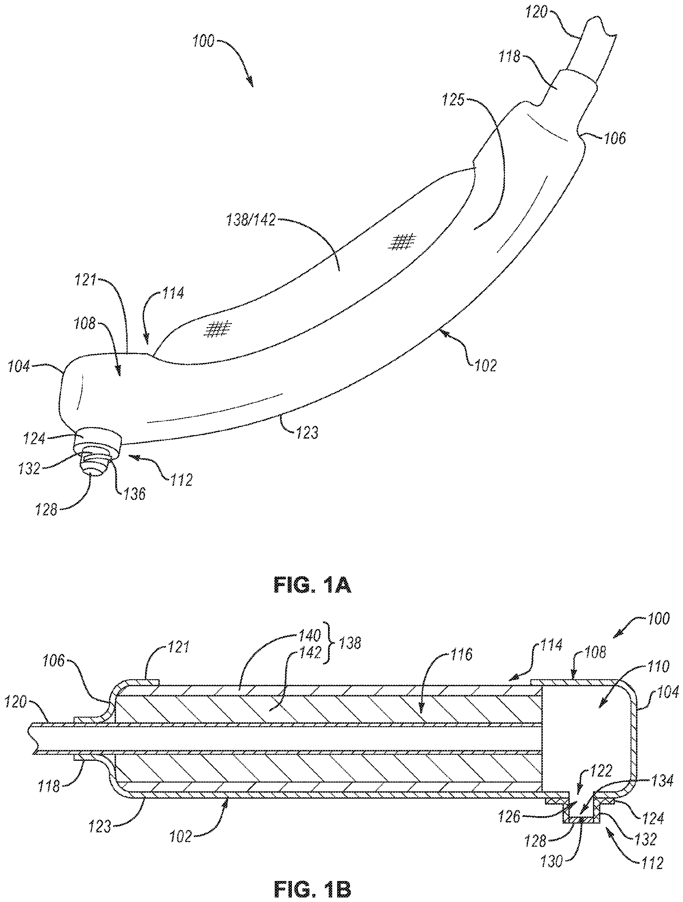

With conventional catheters (e.g., Foley catheters), sample ports are provided on a tube extending between the urethral opening and a drainage bag. Samples may be obtained from these sample ports by connecting a fluid collector to the port. The fluid collector then removes a sample of the bodily fluids by pulling the bodily fluids flowing in the tube or by kinking the tube followed by collecting the pooled bodily fluids. However, such methods of obtaining samples from the fluid collection assemblies according to any of the embodiments of the present disclosure (e.g., fluid collection assemblies 100, 200, 300a, 300b, 400a, 400b, 400c, 500 and 600 shown in FIGS. 1 A-6) can be difficult and/or impossible for at least one of several reasons. For example, the fluid collection assemblies according to any of the embodiments of the present disclosure remove the bodily fluids from the fluid reservoir by applying a vacuum force from a tube. The suction force tends to move the fluid through the tube at relatively high velocities which makes removing of the bodily fluids flowing in the tube difficult. Further, kinking the tube to pool the bodily fluids does not cause the bodily fluids to pool in the tube since kinking the tube stops the suction force that pulls the bodily fluids into the tube.

The sample ports according to any embodiments of the present disclosure remedy at least some of the problems associated with obtaining samples of the bodily fluids using conventional sample ports. For example, the sample ports according to any of the embodiments of the present disclosure extend from the sump of the fluid reservoir instead of located on a portion of the tube. The bodily fluids that are present in the fluid reservoir tend to flow at lower velocities (e.g., are more stationary) that the bodily fluids that flow through the tube. Further, the bodily fluids flow towards the fluid reservoir even in the absence of the suction force since, for example, the fluid reservoir is the gravimetric low point of the fluid collection assembly. As such, the suction force that removes the bodily fluids from the fluid reservoir may be stopped (e.g. , the tube is kinked) thereby causing the bodily fluids to pool in the fluid reservoir.

FIG.1 A is a schematic isometric view of a fluid collection assembly 100, according to an embodiment. FIG. 1B is a cross-sectional view of the fluid collection assembly 100, according to an embodiment The fluid collection assembly 100 includes a fluid impermeable barrier 102. The fluid impermeable barrier 102 includes a first end region 104 and a second end region 106 at an opposite end of the fluid collection assembly 100. The fluid impermeable barrier 102 also includes a sump 108 that forms at least a portion of the first end region 104. The sump 108 defines at least a portion of a fluid reservoir 110. The fluid collection assembly 100 also includes a sample port 112 extending from the sump 108. As such, the sample port 112 is in fluid communication with the fluid reservoir 110 and allows a sample to be taken of any of the bodily fluids that are present in the fluid reservoir 110.

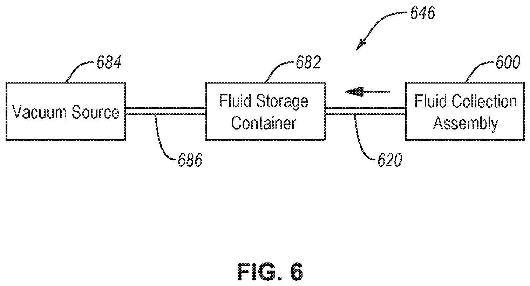

The fluid impermeable barrier 102 defines at least one opening 114, such as a single elongated opening. The opening 114 is located between the first end region 104 and the second end region 106 of the fluid impermeable barrier 102. The opening 114 is configured to be positioned adjacent to a female urethral opening or a buried penis. The fluid impermeable barrier 102 also defines a chamber 116 that is in fluid communication with the opening 114 such that bodily fluids that are discharged by the urethral opening flow through the opening 114 and into the chamber 116. As previously discussed, the bodily fluids that are present in the chamber 116 generally flow towards the fluid reservoir 110. Additionally, the fluid impermeable barrier 102 defines a fluid outlet 118 that allows the bodily fluids to be removed from the fluid reservoir 110. In the illustrated embodiment, the fluid outlet 118 is located on the second end region 106 of the fluid impermeable barrier 102 while the fluid reservoir 110 is located at or near the first end region 104 of the fluid impermeable barrier 102. As such, the fluid collection assembly 100 includes at least one tube 120 positioned in the fluid outlet 118 that extends from the fluid outlet 118 to the fluid reservoir 110 thereby allowing the fluid outlet 118 to be in fluid communication with the fluid reservoir 110. The tube 120 extends from the fluid outlet 118 to another component of a fluid collection system, as will be discussed in more detail with regards to **FIG. 6****.**

The fluid impermeable barrier 102 may be formed of any suitable material. For example, the fluid impermeable barrier 102 may be formed from silicone, another thermosetting polymer, another suitable fluid impermeable material, or combinations thereof. In an embodiment, as illustrated in **FIGS. 1A** and **1B****,** the fluid impermeable barrier 102 may be formed from a single piece. In an embodiment, the fluid impermeable barrier 102 may be formed from a plurality of pieces.

As previously discussed, the fluid impermeable barrier 102 includes a sump 108. The sump 108 may form the first end region 104 of the fluid impermeable barrier 102. The sump 108 includes portions of the fluid impermeable barrier 102 that at least one of defines the fluid reservoir 110 (shown in **FIG. 1B**), extends from the opening 114 to the first end region 104 of the fluid impermeable barrier 102, or is directly attached to the sample port 112. The sump 108 may be integrally formed with the rest of the fluid impermeable barrier 102 or may be formed separately from and attached to the rest of the fluid impermeable barrier 102.

At least a portion of the sump 108 may be at least partially transparent (e.g., translucent or transparent). For example, as previously discussed, the sump 108 defines the fluid reservoir 110 of the fluid collection assembly 100. Selecting at least a portion of the sump 108 to be at least partially transparent allows a user (e.g., medical practitioner) to determine when bodily fluids are present in the fluid reservoir 110. As such, the at least partially transparent portions of the sump 108 allows the user to determine when samples of the bodily fluids may be taken. In an embodiment, the at least partially transparent portion of the sump 108 may also include one or more markings (not shown) thereon. The markings formed on the sump 108 may indicate the volume of the bodily fluids that are present in the fluid reservoir 110. In an example, the markings quantitatively (*e.g.*, in milliliters, fluid ounces, etc.) indicate the volume of the bodily fluids that are present in the fluid reservoir 110. In an example, the markings qualitatively indicate the bodily fluids that are present in the fluid reservoir. In such an embodiment, the markings may indicate when a sufficient quantity of fluids are present in the fluid reservoir 110 to obtain a sample. Examples of transparent material that may form at least a portion of the sump 108 include glass, one or more transparent polymers (*e.g.*, polycarbonate, acrylic, polyethylene terephthalate, polyethylene, etc.), or combinations thereof. In an embodiment, at least a portion of the sump 108 may be at least partially opaque to minimize patient embarrassment.

As previously discussed, the sample port 112 is attached to the sump 108. For example, the sump 108 may define an hole 122 that is adjacent to the fluid reservoir 110. The sample port 112 is attached to the portions of the sump 108 about the hole 122. This allows the sample port 112 to be in fluid communication with the fluid reservoir 110 via the hole 122. Generally, the hole 122 is located at or near the first end region 104 of the fluid impermeable barrier 102 since the first end region 104 is generally at or near the gravimetric low point of the fluid collection assembly 100 during use of the fluid collection assembly 100. As such, even in the absence of a suction force, any bodily fluids that enter the chamber 116 are drawn towards the fluid reservoir 110 and the hole 122.

In an embodiment, as illustrated, the hole 122 is located near (but not at) the first end region 104. Instead, the hole 122 is located on a back surface 123 of the fluid impermeable barrier 102. The back surface 123 of the fluid impermeable barrier 102 is a surface of the fluid impermeable barrier 102 that is opposite or generally opposite the opening 114 defined by the fluid impermeable barrier 102. Forming the hole 122 on the back surface 123 of the fluid impermeable barrier 102 may facilitate obtaining a sample more effectively than if the hole 122 was formed on another surface of the fluid impermeable barrier 102. For example, during operation, the opening 114 of the fluid impermeable barrier 102 is positioned adjacent to the urethral opening of the individual. As such, any front surface 121 of the fluid impermeable barrier 102 (*i.e*., any surface of the fluid impermeable barrier 102 that faces the same direction as the opening 114) is likewise positioned adjacent to the urethral opening, mons pubis, or perineal regions of the individual thereby making access to the front surface 121, for the purpose of obtaining a sample, difficult. Further, any side surface 125 of the fluid impermeable barrier 102 (*i.e.*, any surface of the fluid impermeable barrier 202 between the front surface 121 and the back surface 123) is likely to be positioned adjacent or proximate to the thighs of the individual making access to these surface with a fluid collector difficult. Additionally, the first end region 104 of the fluid impermeable barrier 102 may be positioned adjacent to or proximate to a bed when the individual using the fluid collection assembly 100 is lying down thereby making it difficult to access the first end region 104 with a fluid collector. Meanwhile, the back surface 123 of the fluid impermeable barrier 102 may be easily accessible with a fluid collector, especially if the individual using the fluid collection assembly 100 opens their legs slightly. However, it is noted that the hole 122 may be located on any suitable surface of the sump 108, such as at the first end region 104, on the front surface 121 of the sump 108, or on a side surface 125 of the sump 108, without limitation.

The sample port 112 includes an attachment portion 124 that is configured to be attached to the portions of the sump 108 that are around the hole 122. The attachment portion 124 may be attached to the sump 108 using any suitable technique. In an embodiment, the attachment portion 124 is attached to the sump 108 using ultrasonic welding which forms molecular bonds or other strong attachment forces between the attachment portion 124 and the sump 108. However, in some embodiments, the attachment portion 124 may only be attached to the sump 108 using ultrasonic welding when both the attachment portion 124 and the sump 108 are thermoplastic and both the attachment portion 124 and the sump 108 are compatible, which is not always the case. In an embodiment, the attachment portion 124 is configured to be attached to the sump 108 via heat staking. In such an embodiment, the attachment portion 124 is attached to the fluid impermeable barrier 102 via heat staking. In such an embodiment, one of the attachment portion 124 or the portion of the sump 108 that is configured to be attached to the attachment portion 124 includes one or more protrusions while the other of the attachment portion 124 or the portion of the sump 108 that is configured to be attached to the attachment portion 124 defines passageways extending therethrough that are configured to receive respectively ones of the one or more protrusions. After positioning the protrusions through the passageways, pressure and heat are applied to the protrusions which deforms the protrusions into "stakes" that exhibit a general mushroom-like shape. The stakes are then cooled such that the stakes maintain the shape thereof. One benefit of attaching the attachment portion 124 to the sump 108 using heat staking is that the materials that form the attachment portion 124 and the sump 108 do not have to be compatible (unlike ultrasonic welding) and only one of the attachment portion 124 or the sump 108 have to be a thermoplastic material. As such, heat staking may be used to attach the attachment portion 124 to the sump 108 when the attachment portion 124 and the sump 108 include dissimilar materials, though heat staking may also be used when the attachment portion 124 and the sump 108 include similar materials. In an embodiment, the attachment portion 124 may be attached to the sump 108 using other suitable techniques, such as with an adhesive.

In an embodiment, the attachment portion 124 defines a port opening 126 that allows the bodily fluids to flow through the hole 122 and into/through the sample port 112. For example, the attachment portion 124 may exhibit a generally annular shape or other similar shape which may provide a surface to be attached to the sump 108 in a fluid tight manner while also defining the port opening 126.

The sample port 112 includes a valve 128. As used herein, the valve 128 includes any suitable mechanism that is configured to switch between a closed state that restricts fluid flow therethrough and an open state that permits fluid flow therethrough. In an embodiment, the valve 128 may be a split septum fitting that includes a deformable flexible material defining an gap 130 (*e.g*., slit, pin hole, etc.). The valve 128 may be configured such that the gap 130 is sufficiently small that fluid cannot flow therethrough (*e.g*., the gap 130 is defined by a hydrophobic material) when the valve 128 is in a relaxed state (*i.e.,* closed state). However, pressing against the valve 128 with, for example, a fluid collector causes the flexible material of the valve 128 to deform and increase the size of the gap 130 such that fluid may flow through the gap 130. Removing the pressure applied to the valve 128 may cause the gap 130 to decrease in size such that the gap 130 again exhibits a size sufficiently small that fluid cannot flow therethrough. In an embodiment, the valve 128 includes a plastic layer (*e.g*., a plastic membrane) extending across an opening thereof (*e.g*., the port opening 126). The plastic layer may not initially define a gap. However, a syringe may easily penetrate the plastic layer to form a gap. However, the gap may exhibit a size, after removal of the syringe, that is sufficiently small that fluid cannot flow therethrough (*e.g*., the plastic layer is formed of a hydrophobic material). In an embodiment, the valve 128 may include a mechanical valve that switches between the open and closed states thereof responsive to actuation of an actuator. For example, the valve 128 may include a butterfly valve, a ball valve, or any other suitable mechanical valve.

In an embodiment, the valve 128 is spaced from the attachment portion 124. In such an embodiment, the sample port 112 may include at least one wall 132 extending from the attachment portion 124 to the valve 128. The wall 132 may define a passageway 134 extending from the port opening 126 to the valve 128. The wall 132 may facilitate attachment of a fluid collector to the sample port 112. In an example, the wall 132 may include one or more threads 136 formed therein. The threads 136 may secure the fluid collector to the sample port 112 while taking a sample. The threads 136 may also allow the connection device to be connected to the sample port 112 by twisting (*e.g.*, without applying a pressure to the sump 108) which may prevent contraction of the sump 108. In an example, the wall 132 allows the fluid collector to be attached to the sample port 112 using a luer lock mechanism.

The fluid collection assembly 100 includes at least one wicking material 138 disposed in the chamber 116. In the illustrated embodiment, referring to **FIG. 1B****,** the wicking material 138 includes a fluid permeable support 140 and a fluid permeable membrane 142. The fluid reservoir 110, the fluid permeable support 140, and the fluid permeable membrane 142 may be arranged such that any bodily fluids flowing through the opening 114 flows through the fluid permeable membrane 142, through the fluid permeable support 140, and into the fluid reservoir 110. The bodily fluids may then flow from the fluid reservoir 110 to the tube 120 thereby removing the bodily fluids from the chamber 116.

The fluid permeable support 140 can be positioned relative to the permeable membrane 142 such that the fluid permeable support 140 maintains the permeable membrane 142 in a particular shape. In an embodiment, the fluid permeable support 140 can be configured to maintain the permeable membrane 142 against or near the urethral opening of the individual. For example, the fluid permeable support 140 can include a portion having a curved shape in contact with the permeable membrane 142 such that the permeable membrane 142 is also curved, thus creating a comfortable and secure interface for engagement with the urethral opening and/or the area of the body near the urethral opening.

In an embodiment, the fluid permeable support 140 can be made of a rigid plastic. In an embodiment, the fluid permeable support 140 can have any suitable shape and be formed of any suitable material. For example, the fluid permeable support 140 can be flexible. Additionally, the fluid permeable support 140 can be formed of aluminum, a composite of plastic and aluminum, some other metal and/or a composite. In an embodiment, the fluid permeable support 140 can be formed of a natural material, such as, for example, plant fibers (e.g., Greener Clean manufactured by 3M^{®}). The natural material can include openings that allow fluid to flow through the natural material. In an embodiment, the fluid permeable support 140 can be cylindrical and can define a lumen. In an embodiment, the fluid permeable support 140 can be formed of perforated coated paper, such as tubular waxed paper. In an embodiment, the fluid permeable support 140 may be formed from spun plastic, such as non-woven permeable nylon and/or polyester webbing.

The fluid permeable membrane 142 can be formed of a material that is permeable to the bodily fluids and has wicking properties. The fluid permeable membrane 142 can have a high absorptive rate and a high permeation rate such that the bodily fluids can be rapidly absorbed by the fluid permeable membrane 142 and/or transported through the fluid permeable membrane 142. In an embodiment, the fluid permeable membrane 142 can be a ribbed knit fabric. In an embodiment, the fluid permeable membrane 142 can include and/or have the moisture-wicking characteristic of gauze, felt, terry cloth, thick tissue paper, and/or a paper towel. In an embodiment, the fluid permeable membrane 142 can be soft and/or minimally abrasive such that the fluid permeable membrane 142 does not irritate the skin of the user. The fluid permeable membrane 142 can be configured to wick bodily fluids away from the urethral opening and/or the skin of the individual such that the dampness of the skin of the individual is lessened and infections are prevented. Additionally, the wicking properties of the fluid permeable membrane 142 can help prevent bodily fluids from leaking or flowing beyond the assembly onto, for example, a bed. In an embodiment, the fluid permeable membrane 142 can be formed of fine denier polyester fibers coated with a thermoplastic water-based binder system.

In an embodiment, the at least one of the fluid permeable support 140 or the fluid permeable membrane 142 may be omitted from the fluid collection assembly 100 such that the wicking material 138 only includes a single material. In an embodiment, the wicking material 138 may include three or more materials, such as the fluid permeable support 140, the fluid permeable membrane 142, and at least one additional material. Regardless, one or more components of the wicking material 138 may include permeable material designed to wick or pass fluid therethrough. The permeable properties referred to herein may be wicking, capillary action, diffusion, or other similar properties or processes, and are referred to herein as "permeable" and/or "wicking." Such "wicking" may not include absorption of one or more components of the bodily fluids (*e.g*., urine) into the one or more components of the wicking material 138. Put another way, substantially no absorption of one or more components of the bodily fluids into the one or more components of the wicking material 138 may take place after the wicking material 138 is exposed to the bodily fluids. While no absorption is desired, the term "substantially no absorption" may allow for nominal amounts of absorption of one or more components of the bodily fluids into the wicking material 138 (*e.g*., absorbency), such as less than about 10 wt% of the dry weight of the wicking material 138, less than about 7 wt%, less than about 5 wt%, less than about 3 wt%, less than about 2 wt%, less than about 1 wt%, or less than about 0.5 wt% of the dry weight of the wicking material 138. In an embodiment, the fluid collection assembly 100 may include one or more absorbent or adsorbent materials instead of or in addition to the wicking material 138.

Additional examples of fluid collection assemblies are disclosed in U.S. Patent No. 10,226,376.

As will be discussed in more detail with regards to FIG. 6, the fluid collection assembly 100 may be in fluid communication with a fluid storage container and a vacuum source. For example, the fluid outlet 118 may be coupled to at least one tube 120 and the tube 120 may fluidly couple the fluid collection assembly 100 to the fluid storage container and the vacuum source. The tube 120 may also extend behind the wicking material 138 such that the tube 120 is positioned in or near the fluid reservoir 110.

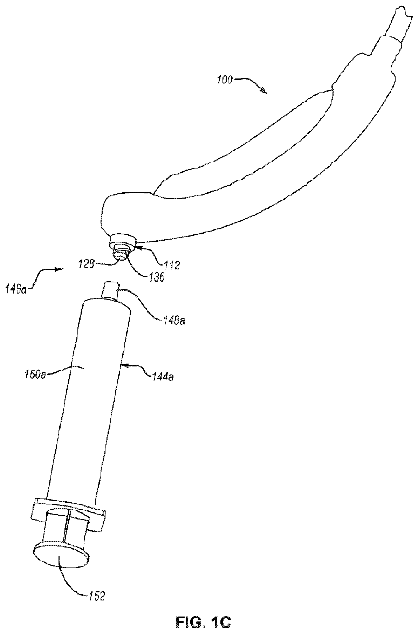

The fluid collection assembly 100 may form part of a fluid collection system that includes at least one fluid collector. For example, FIGS. 1C and ID are isometric views of a portion of different fluid collection systems, according to different embodiments. The fluid collection systems illustrated in FIGS. 1C and ID include the fluid collection assembly 100 and different fluid collectors. Although FIGS. 1C and ID illustrate fluid collection systems including the fluid collection assembly 100 illustrated in FIGS. 1A and 1B, it is noted that the illustrated fluid collection systems may include any of the fluid collection assemblies disclosed herein.

Referring to FIG. 1C, the fluid collector 144a of the fluid collection system 146a includes a needleless syringe that is configured to connect to the sample port 112. For example, the fluid collector 114a includes a collector port 148a that is configured to connect with the sample port 112. For example, the collector port 148a may connect with the sample port 112 in a fluid tight manner. The collector port 148a may also be configured to be secured to the sample port 112 to prevent inadvertently disconnecting the collector port 148a from the sample port 112. For example, the collector port 148a may include one or more threads (not shown, obscured) that are configured to mate with the threads 136 of the sample port 112. In an embodiment, the sample port 112 and the collector port 148a may form a luer-lock connection. In an embodiment, the collector port 148a may be configured to switch the valve 128 of the sample port 112 from the closed state thereof to the open state thereof when the collector port 148a is connected to the sample port 112.

The fluid collector 144a may also include a fluid reservoir 150a that is in fluid communication with the collector port 148a. For example, the fluid reservoir 150a may receive any bodily fluids that are received by the collector port 148a. The fluid collector 144a may also include a plunger 152 connected to the fluid reservoir 150a. The plunger 152 may be configured to selectively pull bodily fluids through the collector port 148a and into the fluid reservoir 150a and/or push the bodily fluids from the fluid reservoir 150a and through the collector port 148a responsive to manipulation of the plunger 152 by an user of the fluid collector 144a.

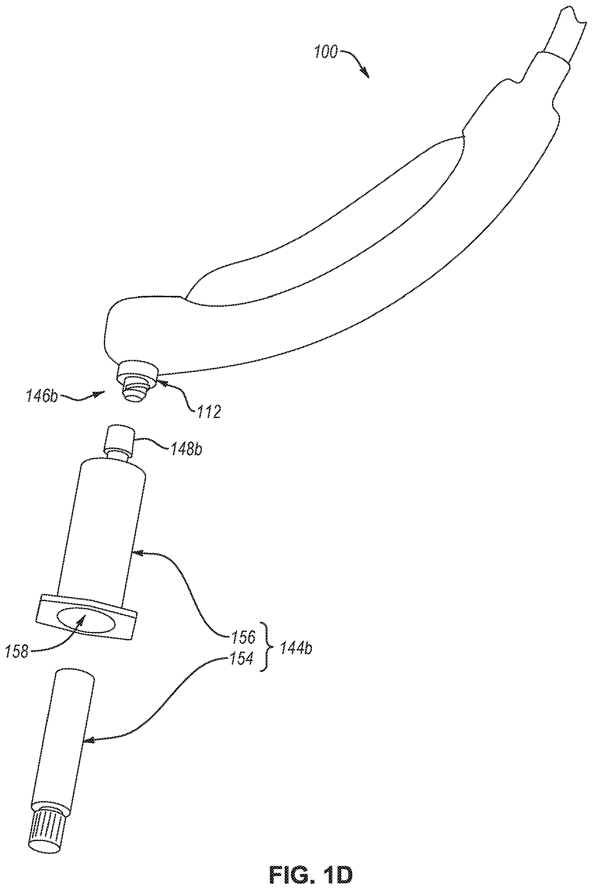

Referring to **FIG. 1D****,** the fluid collector 144b of the fluid collection system 146b includes a vial 154. The vial 154 may be initially under vacuum such that connecting the vial 154 to the sample port 112 pulls any bodily fluids from the fluid reservoir 110 of the fluid collection assembly 100 into the vial 154. In an embodiment, not shown, the vial 154 is configured to be directly connected to the sample port 112. In an embodiment, as illustrated, the vial 154 is configured to be indirectly connected to the sample port 112 using an intermediary device 156. The intermediary device 156 may include a collector port 148b that is the same or similar to the collector port 148b. The intermediary device 156 may also define a receptacle 158 that is configured to receive at least a portion of the vial 154.

It is noted that the fluid collection systems disclosed herein may be used with fluid collectors other than the fluid collectors illustrated in **FIGS. 1C** and **1D****.** For example, the fluid collection systems disclosed herein may include a needle syringe or any other suitable fluid collector.

Referring to **FIGS. 1A** to **1D****,** during use, the fluid collection assembly 100 is positioned such that the opening 114 is adjacent to the urethral opening of an individual. The fluid collection assembly 100 may then receive bodily fluids from the urethral opening of the individual through the opening, into the chamber 116 (*e.g*., into the at least one wicking material 138), and to the fluid reservoir 110. The bodily fluids may then be removed from the fluid reservoir 110 through the tube 120 using a suction force. Samples of the bodily fluids may be obtain by connecting a fluid collector (*e.g.*, fluid collector 144a or fluid collector 144b) and extracting the bodily fluids from the fluid reservoir 110 into the fluid collector. In an embodiment, the sample of the bodily fluids may be extracted from the fluid reservoir 110 while a suction force is applied from the tube 120 to the fluid reservoir 110. However, the suction force that is applied from the tube 120 to the fluid reservoir 110 may limit the quantity of bodily fluids that are obtained. As such, in an embodiment, the suction force is turned off at least while the sample is extracted from the fluid reservoir 110. The suction force may be turned off by kinking the tube 120, turning off the vacuum source, etc. Turning off the suction force may allow the bodily fluids to pool in the fluid reservoir and may prevent the removal of the bodily fluids until after the sample is obtained.

FIG. 2 has been excised from this specification.

The fluid reservoir of the fluid collection assemblies disclosed herein are configured to hold bodily fluids therein until the bodily fluids are removed using a suction force from a tube. Generally, the suction force from the tube removes the bodily fluids quickly from the fluid reservoir. However, kinking the tube or otherwise stopping the suction force may cause the bodily fluids to pool in the fluid reservoir. Thus, in some embodiments, the fluid reservoir disclosed herein may need to exhibit a volume that is greater than similar conventional fluid collection assemblies to prevent overfilling the fluid reservoir since overfilling the fluid reservoir may cause the bodily fluids to leak from the chamber. For example, the fluid reservoirs of the fluid collection assemblies disclosed herein may exhibit a volume that is greater than 15 milliliters ("mL"), greater than about 30 mL, greater than about 50 mL, greater than about 75 mL, greater than about 100 mL, greater than about 150 mL, greater than about 200 mL, greater than about 250 mL, greater than about 300 mL, greater than about 400 mL, greater than about 500 mL, greater than about 600 mL, greater than about 700 mL, greater than about 800 mL, or in ranges of about 15 mL to about 50 mL, about 30 mL to about 75 mL, about 50 mL to about 100 mL, about 75 mL to about 150 mL, about 100 mL to about 200 mL, about 150 mL to about 250 mL, about 200 mL to about 300 mL, about 250 mL to about 400 mL, about 300 mL to about 500 mL, about 400 mL to about 600 mL, about 500 mL to about 700 mL, or about 600 mL to about 800 mL.

The volume of the fluid reservoir may depend on a number of factors. In an example, the volume of the fluid reservoir may depend on whether the individual using the fluid collection assembly can control urination. For instance, an individual who has no control over urination may urinate more often than an individual who has control over urination. As such, the volume of a fluid reservoir may be smaller (*e.g*., less than 400 mL, less than 300 mL, or less than 200 mL) for an individual who has no control over urination than the volume of a fluid reservoir for an individual who has control over urination. It is noted that an individual who has control over urination may still use a fluid collection assembly with a volumetrically small fluid reservoir when the individual is given direction to urinate frequently. In an example, the volume of the fluid reservoir may depend on whether the suction force is expected to be stopped to obtain a sample since not stopping the suction force allows the fluid reservoir to exhibit a relatively small volume. In an example, the volume of the fluid reservoir may be selected based on the expected size of the bladder of the individual. For instance, a child or a smaller individual may use a fluid collection assembly having a relatively small fluid reservoir compared to larger individuals. In an example, as previously discussed, the size of the fluid reservoir may depend on whether the sample port includes a tube and the relative length of the tube.

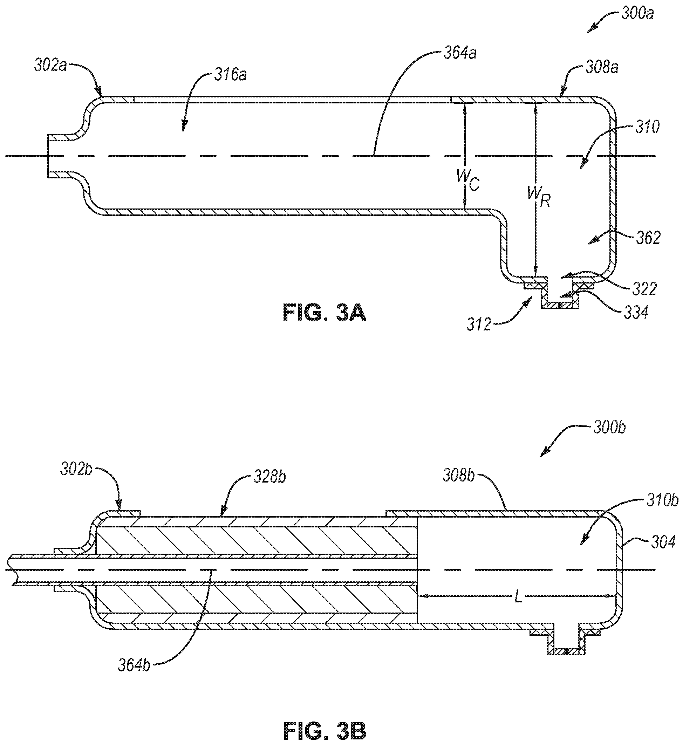

The fluid collection assemblies may exhibit a variety of different structures that allow the fluid reservoirs to exhibit the volumes disclosed herein. For example, **FIG. 3A** is a schematic cross-sectional view of a fluid collection assembly 300a, according to an embodiment. Except as otherwise disclosed herein, the fluid collection assembly 300a is the same or substantially similar to any of the fluid collection assemblies disclosed herein. For example, the fluid collection assembly 300a includes a fluid impermeable barrier 302a defining a chamber 316a. For clarity, the chamber 316a is illustrated as being empty. However, it is noted that one or more elements may be disposed in the chamber 316a, such as at least one wicking material.

The fluid impermeable barrier 302a includes a sump 308a defining a fluid reservoir 310a. The sump 308a defines a bulge 362 which increases the volume of the fluid reservoir 310a. For example, the bulge 362 may cause the fluid reservoir 310a defined to exhibit a maximum reservoir width W_{R} that is measured perpendicular to the longitudinal axis 364a of the fluid impermeable barrier 302a. It is noted that the maximum reservoir width W_{R} does not extend into the hole 322 defined by the fluid impermeable barrier 302a or a passageway 334 defined by the sample port 312. The maximum reservoir width W_{R} is greater than a maximum chamber width W_{C} of the chamber 316a, wherein the maximum chamber width W_{C} is measured at a location that is spaced from the fluid reservoir 310a. The greater width of the maximum reservoir width W_{R} relative to the maximum chamber width W_{C} increases the volume of the fluid reservoir 310a than if the fluid impermeable barrier 302a did not include the bulge 362.

In an embodiment, the maximum reservoir width W_{R} may be greater than the maximum chamber width W_{C} by at least a factor of about 1.25, at least a factor of about 1.5, at least a factor of about 1.75, at least a factor of about 2, at least a factor of about 2.5, at least a factor of about 3, at least a factor of about 3.5, at least a factor of about 4, or at least a factor of about 5. In an embodiment, the maximum reservoir width W_{R} may be about 3 cm to about 4 cm, about 3.5 cm to about 4.5 cm, about 4 cm to about 5, about 4.5 cm to about 6 cm, about 5 cm to about 7 cm, about 6 cm to about 8 cm, about 7 cm to about 9 cm, about 8 cm to about 10, about 9 cm to about 11 cm, or greater than 10 cm and the maximum chamber width W_{C} may be about 1 cm to about 3 cm, about 2 cm to about 4 cm, or about 3 cm to about 5 cm. The maximum reservoir width W_{R} and the ratio of the maximum reservoir width W_{R} to the maximum chamber width W_{C} may be selected based on the desired volume of the fluid reservoir 310a.

Instead of increasing the width of the fluid reservoir, in an embodiment, the fluid reservoir may exhibit an elongated length. For example, **FIG. 3B** is a schematic cross-sectional view of a fluid collection assembly 300b having an elongated fluid reservoir 310b, according to an embodiment. Except as otherwise disclosed herein, the fluid collection assembly 300b is the same or substantially to any of the fluid collection assemblies disclosed herein. For example, the fluid collection assembly 300b may include a fluid impermeable barrier 302b and at least one wicking material 338b. The fluid impermeable barrier 302b may include a sump 308b defining the fluid reservoir 310b.

The fluid reservoir 310b (*e.g.*, a substantially unoccupied fluid reservoir 310b) may exhibit a maximum length L. The maximum length L may be measured from the at least one wicking material 338b to the first end region 304 of the fluid impermeable barrier 302b. For example, the maximum L of the fluid reservoir 310b may be measured parallel to a longitudinal axis 364b of the of the fluid impermeable barrier 302b. Increasing the maximum length L of the fluid reservoir 310b increases the volume of the fluid reservoir 310b thereby increasing the quantity of bodily fluids that may be stored in the fluid reservoir 310b.

The maximum length L of the fluid reservoir 310b may be greater than the length of fluid reservoirs of conventional fluid collection assemblies. In an embodiment, the maximum length L may be greater than about 3 cm, greater than about 4 cm, greater than about 5 cm, greater than about 6 cm, greater than about 7 cm, greater than about 8 cm, greater than about 9 cm, greater than about 10 cm, greater than about 12.5 cm, greater than about 15 cm, or in ranges of about 3 cm to about 5 cm, about 4 cm to about 6 cm, about 5 cm to about 7 cm, about 6 cm to about 8 cm, about 7 cm to about 9 cm, about 8 cm to about 10 cm, about 9 cm to about 12.5 cm, or about 10 cm to about 15 cm. The maximum length L of the fluid reservoir 310b may be selected based on the desired volume of the fluid reservoir 310b.

As previously discussed, the fluid impermeable barriers disclosed herein may be at least partially formed from silicone or another easily deformable material. Such fluid impermeable barriers allow the fluid collection assemblies disclosed herein to be fitted to an individual. For example, the curvature and topography of the region about the urethral opening may be different for different individuals. The easily deformable fluid impermeable barrier allows the fluid collection assembly to easy conform to the curvature and topography of each individual. However, the easily deformable material of the fluid impermeable barrier may inadvertently deform when a fluid collector is connected to the sample port. When bodily fluids are present in the fluid reservoir, inadvertently deforming the fluid impermeable barrier may decrease the volume of the fluid reservoir and cause the bodily fluids to leak from the chamber.

In an embodiment, the leakage caused by connecting the fluid collector to the sample port may be mitigated by connecting the fluid collector to the sample port before bodily fluids are present in the fluid reservoir (*e.g*., before stopping the suction force). In an embodiment, the inadvertent deformation may be mitigated by using threads to connect the fluid collector to the sample port since the force required to tread the fluid collector onto the sample port, if performed properly, may only deform the fluid impermeable barrier an insignificant amount. In an embodiment, the attachment portion of the sample port may exhibit a large diameter (*e.g*., may extend radially outwardly from the walls of the sample port) that mitigates any deformation by spreading any force caused by connecting the fluid collector to the sample port over a larger area of the fluid impermeable barrier.

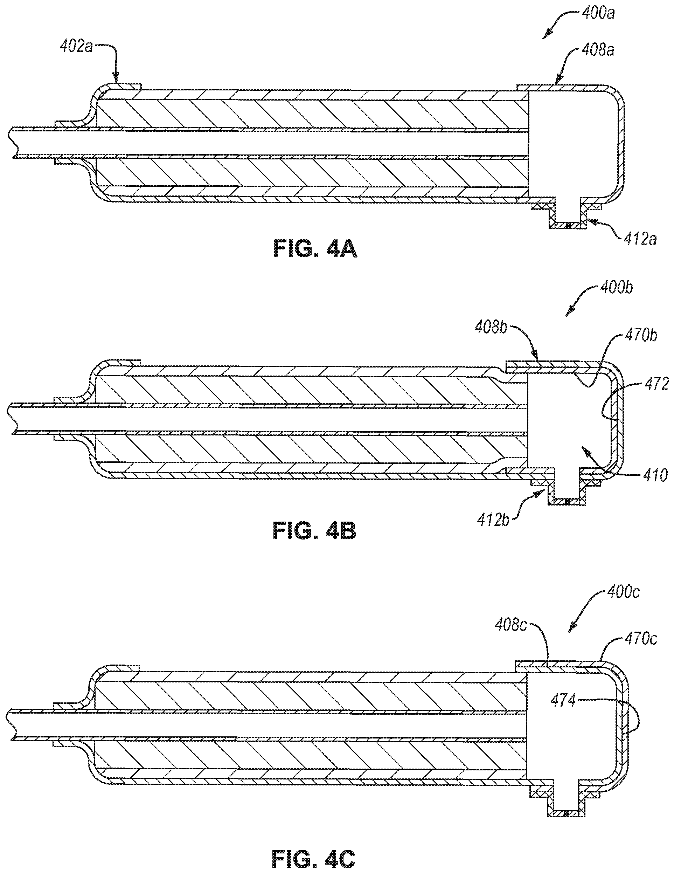

In an embodiment, the inadvertent deformation of the fluid impermeable barrier may be mitigated by increasing the rigidity of the sump relative to the rest of the fluid impermeable barrier. For example, **FIGS. 4A** to **4C** are schematic cross-sectional views of fluid collection assemblies including sumps exhibiting a greater rigidity (*i.e.,* better to resist deformations) than the rest of the fluid impermeable barriers thereof, according to different embodiments. Except as otherwise disclosed herein, the fluid collection assemblies illustrated in **FIGS. 4A-4C** are the same or substantially similar to any of the fluid collection assemblies disclosed herein.

Referring to **FIG. 4A****,** the sump 408a itself (*i.e.,* without any support structure) exhibits a rigidity that is greater than the rest of the fluid impermeable barrier 402a of the fluid collection assembly 400a. For example, the sump 408a may be distinct from the rest of the fluid impermeable barrier 402a. In an embodiment, as illustrated, the sump 408a may be formed from a material that is different than and exhibits an elasticity (*i.e*., Young's modulus) that is greater than the rest of the fluid impermeable barrier 402a. The increased elasticity of the sump 408a may allow the sump 408a to better resist deformation when the fluid collector is attached to the sample port 412a. Examples of material that may form the sump 408a and may exhibit an elasticity greater than the rest of the fluid impermeable barrier 402a includes epoxy, polyacetal, polyester, metal, or any other suitable material. Since the sump 408a is formed from a different material than the rest of fluid impermeable barrier 402a, the sump 408a may be attached to the rest of the fluid impermeable barrier 402a using any suitable technique, such as with an adhesive, ultrasonic welding, heat staking, etc. In an embodiment, the sump 408a may exhibit a thickness that is greater than the rest of the fluid impermeable barrier 402a. The increased thickness of the sump 408a may increase the rigidity of the sump 408a and may allow the sump 408a to better resist deformation.

Referring to **FIG. 4B****,** the fluid collection assembly 400b may include a support structure 470b that is configured to increase the rigidity of the sump 408b. The support structure 470b may exhibit a size that allows the support structure 470b to be disposed adjacent to an interior surface 472 of the sump 408b. In other words, the support structure 470b may be configured to be disposed in at least the fluid reservoir 410 and/or at least a portion of the support structure 470b exhibits a shape the corresponds to the shape of the interior surface 472 of the sump 408b. In an embodiment, the support structure 470b may be adjacent to an entirety of the interior surface 472 of the sump 408b or adjacent to only a portion of the interior surface 472 of the sump 408b (*e.g*., adjacent to the portion of the interior surface 472 that is proximate to the sample port 412b). In an embodiment, the support structure 470b may be adjacent to portions of the fluid impermeable barrier 402b that do not form part of the sump 408b in addition to being adjacent to at least a portion of the interior surface 472 of the sump 408b.

The support structure 470b is configured to support the sump 408b thereby increasing the rigidity of the sump 408b. In an embodiment, the support structure 470b is formed from a material exhibiting an elasticity (i.e., Young's modulus) that is greater than the sump 408b. For example, the support structure 470b may include epoxy, polyacetal, polyester, metal, or any other suitable material. In an embodiment, the support structure 470b is formed from a material exhibiting an elasticity that is equal to or less than the sump 408b since even such support structures increase the rigidity of the sump 408. In an embodiment, the support structure 470b is a solid plate (*e.g*., bent or molded plate). In an embodiment, the support structure 470b is a mesh since the mesh may be lighter than the solid plate which make the fluid collection assembly 400b easier to use without significantly decreasing the rigidity of the support structure 470b.

Disposing the support structure 470b in the fluid reservoir 410b may prevent the support structure 470b from coming in contact with an individual using the fluid collection assembly 400b which may make the fluid collection assembly 400b more comfortable to use. However, the presence of the support structure 470b in the fluid reservoir 410b may at least one of decrease the volume of the fluid reservoir 410b, compress the at least one wicking material 438, or require the at least one wicking material 438 to exhibit a non-cylindrical shape. As such, referring to **FIG. 4C****,** is may be beneficial to form a fluid collection assembly 400c that includes a support structure 470c on at least a portion of an exterior surface 474 of the sump 408c. The support structure 470c may be the same as the support structure 470b of **FIG. 4C** except that the support structure 470c exhibits a shape that corresponds to at least a portion of the exterior surface 474. Further, the support structure 470c may include a coating (*e.g.*, a silicone coating) that makes the support structure 470c more comfortable against the individual during use.

The embodiments disclosed above are for fluid collection assemblies that are configured to receive bodily fluids from a female urethral opening or a buried penis.

**FIG.6** is a schematic illustration of fluid collection system 680 that includes a fluid collection assembly 600, according to an embodiment. The fluid collection assembly 600 may include any of the fluid collection assemblies disclosed herein. The fluid collection assembly 600 may be in fluid communication with a fluid storage container 646 via, for example, at least one first tube 620 (e.g., tube 120 of **FIGS. 1A** and **1B**)**.** The fluid storage container 646 is positioned downstream from the fluid collection assembly 600. The fluid storage container 646 may be in fluid communication with a vacuum source 684 via at least one second tube 686. The vacuum source 684 is positioned downstream from the fluid storage container 646. During operation, the vacuum source 684 provides a suction force to the fluid collection assembly 600. The suction force draws fluid into the chamber of the fluid collection assembly 600 and towards the first tube 620. The fluid that enters the first tube 620 is pulled by the suction force towards the fluid storage container 646 such that the fluid storage container 646 receives the bodily fluids. The fluid storage container 646 may be configured to inhibit the fluid from flowing from the fluid storage container 646 to the vacuum source 684.

While various aspects and embodiments have been disclosed herein, other aspects and embodiments are contemplated. The various aspects and embodiments disclosed herein are for purposes of illustration and are not intended to be limiting.

Terms of degree (e.g., "about," "substantially," "generally," etc.) indicate structurally or functionally insignificant variations. In an example, when the term of degree is included with a term indicating quantity, the term of degree is interpreted to mean ± 10%, ±5%, +2% or 0% of the term indicating quantity. In an example, when the term of degree is used to modify a shape, the term of degree indicates that the shape being modified by the term of degree has the appearance of the disclosed shape. For instance, the term of degree may be used to indicate that the shape may have rounded corners instead of sharp corners, curved edges instead of straight edges, one or more protrusions extending therefrom, is oblong, is the same as the disclosed shape, etc.

## Claims

1. A fluid collection assembly (100), comprising:
a fluid impermeable barrier (102) including a first end region (104) and a second end region (106) longitudinally spaced from the first end region, the fluid impermeable barrier including a sump (108) that forms at least a portion of the first end region, the fluid impermeable barrier defining:
at least one opening (114) said opening being located between the first end region and the second end region of the fluid impermeable barrier;
a chamber (116) in fluid communication with the at least one opening;
a fluid reservoir (110) defined by at least a portion of the sump; and
a fluid outlet (118);
the fluid collection assembly further comprising:
at least one wicking material (138) disposed in the chamber;
a tube (120) in fluid communication with the fluid reservoir; and
a sample port (112) in fluid communication with the fluid reservoir, the sample port configured to switch between a closed state that restricts fluid flow through the sample port and an open state that permits fluid flow through the sample port, the sample port configured to be connected to a fluid collector (144b); and wherein:
the fluid outlet (118) is located on the second end region of the fluid impermeable barrier;
the tube (120) is positioned in the fluid outlet (118) and extends from the fluid outlet to the fluid reservoir (110) such that the tube (120) is positioned in or near the fluid reservoir, thereby allowing the fluid outlet (118) to be in fluid communication with the fluid reservoir;
the tube (120) extends from the fluid outlet (118) for connection to another component of the fluid collection system; and wherein
the sample port is attached to the sump (108) of the fluid reservoir and extends from a back surface of the fluid impermeable barrier, wherein the back surface of the fluid impermeable barrier is opposite the at least one opening.

2. The fluid collection assembly of claim 1, wherein the fluid reservoir (110) exhibits a rigidity that is greater than the rest of the fluid impermeable barrier.

3. The fluid collection assembly of claim 2, further comprising a support structure (470b) supporting and providing rigidity to the fluid reservoir (110).

4. The fluid collection assembly of any one of claims 1-3, wherein one or more regions of the sump (108) are at least partially transparent.

5. The fluid collection assembly of any one of claims 1-4, wherein the sump (108) is integrally formed with the rest of the fluid impermeable barrier.

6. The fluid collection assembly of any one of claims 1-5, wherein the fluid reservoir (110) is substantially unoccupied by said wicking material (138) and the fluid reservoir exhibits a length that is greater than about 2.5 cm, wherein the length of the fluid reservoir is measured parallel to a longitudinal axis of the fluid impermeable barrier.

7. The fluid collection assembly of any one of claims 1-6, wherein the sample port (112) is attached to the first end region (104) of the fluid impermeable barrier.

8. The fluid collection assembly of any one of claims 1-7, wherein the sample port (112) is a split septum fitting.

9. The fluid collection assembly of any one of claims 1-8, wherein the sample port (112) forms part of a luer-lock mechanism.

10. The fluid collection assembly of any one of claims 1-9, wherein the sample port (112) is at least one of ultrasonically welded or heat staked to the fluid impermeable barrier.

11. The fluid collection assembly of any one of claims 1-10, wherein the sample port (112) includes a tube.

12. The fluid collection assembly of any one of claims 1-11, wherein the sample port is configured to be in the closed state when the sample port is not attached to the fluid collector and in the open state when the fluid collector is attached to the sample port.

13. A fluid collection system, comprising:
the fluid collection assembly of any one of claims 1-12; and
the fluid collector (646).

14. A method of using the fluid collection system of claim 13, the method comprising:
attaching the fluid collector to the sample port;
receiving bodily fluids from an individual through the at least one opening, into the chamber, and to the fluid reservoir;
removing through the sample port (112) into the fluid collector (144b) at least some of the bodily fluids that are present in the fluid reservoir;
detaching the fluid collector (144b) from the sample port (112);
switching the sample port from the open state to the closed state; and
removing substantially all of a remainder of the bodily fluids present in the fluid reservoir through the fluid outlet (118) using a suction force.

## Patentansprüche

1. Fluidsammelanordnung (100), umfassend:
eine fluidundurchlässige Barriere (102), die einen ersten Endbereich (104) und einen zweiten Endbereich (106) einschließt, der in Längsrichtung vom ersten Endbereich beabstandet ist, wobei die fluidundurchlässige Barriere einen Sumpf (108) einschließt, der mindestens einen Abschnitt des ersten Endbereichs bildet, wobei die fluidundurchlässige Barriere definiert:
mindestens eine Öffnung (114), wobei sich die Öffnung zwischen dem ersten Endbereich und dem zweiten Endbereich der fluidundurchlässigen Barriere befindet;
eine Kammer (116), die in Fluidverbindung mit der mindestens einen Öffnung steht;
ein Fluidreservoir (110), das durch mindestens einen Abschnitt des Sumpfes definiert ist; und
einen Fluidauslass (118);
wobei die Fluidsammelanordnung weiter umfasst:
mindestens ein Dochtwirkungsmaterial (138), das in der Kammer angeordnet ist;
ein Röhrchen (120), das in Fluidverbindung mit dem Fluidreservoir steht; und
einen Probenport (112), der in Fluidverbindung mit dem Fluidreservoir steht, wobei der Probenport so konfiguriert ist, dass er zwischen einem geschlossenen Zustand, der den Fluidstrom durch den Probenport einschränkt, und einem offenen Zustand, der den Fluidstrom durch den Probenport ermöglicht, umschaltet, wobei der Probenport so konfiguriert ist, dass er mit einem Fluidsammler (144b) verbunden ist; und wobei:
der Fluidauslass (118) sich am zweiten Endbereich der fluidundurchlässigen Barriere befindet;
das Röhrchen (120) im Fluidauslass (118) positioniert ist und sich vom Fluidauslass zum Fluidreservoir (110) erstreckt, so dass das Röhrchen (120) im oder in der Nähe des Fluidreservoirs positioniert ist, wodurch ermöglicht wird, dass der Fluidauslass (118) in Fluidverbindung mit dem Fluidreservoir steht;
das Röhrchen (120) sich vom Fluidauslass (118) zur Verbindung mit einer anderen Komponente des Fluidsammelsystems erstreckt; und wobei
der Probenport am Sumpf (108) des Fluidreservoirs angebracht ist und sich von einer hinteren Oberfläche der fluidundurchlässigen Barriere erstreckt, wobei die hintere Oberfläche der fluidundurchlässigen Barriere der mindestens einen Öffnung gegenüberliegt.

2. Fluidsammelanordnung nach Anspruch 1, wobei das Fluidreservoir (110) eine Steifigkeit aufweist, die größer ist als der Rest der fluidundurchlässigen Barriere.

3. Fluidsammelanordnung nach Anspruch 2, weiter umfassend eine Stützstruktur (470b), die das Fluidreservoir (110) stützt und ihm Steifigkeit verleiht.

4. Fluidsammelanordnung nach einem der Ansprüche 1-3, wobei ein oder mehrere Bereiche des Sumpfes (108) zumindest teilweise transparent sind.

5. Fluidsammelanordnung nach einem der Ansprüche 1-4, wobei der Sumpf (108) einstückig mit dem Rest der fluidundurchlässigen Barriere ausgebildet ist.

6. Fluidsammelanordnung nach einem der Ansprüche 1-5, wobei das Fluidreservoir (110) durch das Dochtwirkungsmaterial (138) im Wesentlichen unbelegt ist und das Fluidreservoir eine Länge aufweist, die größer als etwa 2,5 cm ist, wobei die Länge des Fluidreservoirs parallel zu einer Längsachse der fluidundurchlässigen Barriere gemessen wird.

7. Fluidsammelanordnung nach einem der Ansprüche 1-6, wobei der Probenport (112) am ersten Endbereich (104) der fluidundurchlässigen Barriere angebracht ist.

8. Fluidsammelanordnung nach einem der Ansprüche 1-7, wobei der Probenport (112) ein geteiltes Septum-Formstück ist.

9. Fluidsammelanordnung nach einem der Ansprüche 1-8, wobei der Probenport (112) einen Teil eines Luer-Lock-Mechanismus bildet.

10. Fluidsammelanordnung nach einem der Ansprüche 1-9, wobei der Probenport (112) an der fluidundurchlässigen Barriere mindestens eines von ultraschallgeschweißt oder mit Wärme verbunden ist.

11. Fluidsammelanordnung nach einem der Ansprüche 1-10, wobei der Probenport (112) ein Röhrchen einschließt.

12. Fluidsammelanordnung nach einem der Ansprüche 1-11, wobei der Probenport so konfiguriert ist, dass er sich im geschlossenen Zustand befindet, wenn der Probenport nicht an dem Fluidsammler angebracht ist, und im offenen Zustand, wenn der Fluidsammler an dem Probenport angebracht ist.

13. Fluidsammelsystem, umfassend:
die Fluidsammelanordnung nach einem der Ansprüche 1-12; und
den Fluidsammler (646).

14. Verfahren zum Verwenden des Fluidsammelsystems nach Anspruch 13, wobei das Verfahren umfasst:
Anbringen des Fluidsammlers am Probenport;
Aufnehmen von Körperfluiden von einer Person durch die mindestens eine Öffnung in die Kammer und zum Fluidreservoir;
Entfernen von mindestens einem Teil der im Fluidreservoir vorhandenen Körperfluide durch den Probenport (112) in den Fluidsammler (144b);
Lösen des Fluidsammlers (144b) vom Probenport (112);
Umschalten des Probenports vom offenen Zustand in den geschlossenen Zustand; und
Entfernen von im Wesentlichen allem eines Rests der im Fluidreservoir vorhandenen Körperfluide durch den Fluidauslass (118) unter Verwendung einer Saugkraft.

## Revendications

1. Ensemble de collecte de fluide (100), comprenant :
une barrière imperméable aux fluides (102) incluant une première région d'extrémité (104) et une deuxième région d'extrémité (106) espacée longitudinalement de la première région d'extrémité, la barrière imperméable aux fluides incluant un puisard (108) qui forme au moins une partie de la première région d'extrémité, la barrière imperméable aux fluides définissant :
au moins une ouverture (114), ladite ouverture étant située entre la première région d'extrémité et la deuxième région d'extrémité de la barrière imperméable aux fluides ;
une chambre (116) en communication fluidique avec l'au moins une ouverture ;
un réservoir de fluide (110) défini par au moins une partie du puisard ; et
une sortie de fluide (118) ;
l'ensemble de collecte de fluide comprenant en outre :
au moins un matériau à effet de mèche (138) disposé dans la chambre ;
un tube (120) en communication fluidique avec le réservoir de fluide ; et
un orifice d'échantillon (112) en communication fluidique avec le réservoir de fluide, l'orifice d'échantillon étant configuré pour passer d'un état fermé qui restreint un écoulement de fluide à travers l'orifice d'échantillon à un état ouvert qui permet un écoulement de fluide à travers l'orifice d'échantillon, l'orifice d'échantillon étant configuré pour être connecté à un collecteur de fluide (144b) ; et dans lequel :
la sortie de fluide (118) est située sur la deuxième région d'extrémité de la barrière imperméable aux fluides ;
le tube (120) est positionné dans la sortie de fluide (118) et s'étend depuis la sortie de fluide jusqu'au réservoir de fluide (110) de telle sorte que le tube (120) soit positionné dans ou à proximité du réservoir de fluide, permettant ainsi à la sortie de fluide (118) d'être en communication fluidique avec le réservoir de fluide ;
le tube (120) s'étend à partir de la sortie de fluide (118) pour une connexion à un autre composant du système de collecte de fluide ; et dans lequel
l'orifice d'échantillon est fixé au puisard (108) du réservoir de fluide et s'étend à partir d'une surface arrière de la barrière imperméable aux fluides, dans lequel la surface arrière de la barrière imperméable aux fluides est opposée à l'au moins une ouverture.

2. Ensemble de collecte de fluide selon la revendication 1, dans lequel le réservoir de fluide (110) présente une rigidité qui est supérieure à celle du reste de la barrière imperméable aux fluides.

3. Ensemble de collecte de fluide selon la revendication 2, comprenant en outre une structure de support (470b) supportant et conférant de la rigidité au réservoir de fluide (110).

4. Ensemble de collecte de fluide selon l'une quelconque des revendications 1 à 3, dans lequel une ou plusieurs régions du puisard (108) sont au moins partiellement transparentes.

5. Ensemble de collecte de fluide selon l'une quelconque des revendications 1 à 4, dans lequel le puisard (108) est formé intégralement avec le reste de la barrière imperméable aux fluides.

6. Ensemble de collecte de fluide selon l'une quelconque des revendications 1 à 5, dans lequel le réservoir de fluide (110) est sensiblement inoccupé par ledit matériau à effet de mèche (138) et le réservoir de fluide présente une longueur qui est supérieure à environ 2,5 cm, dans lequel la longueur du réservoir de fluide est mesurée parallèlement à un axe longitudinal de la barrière imperméable aux fluides.

7. Ensemble de collecte de fluide selon l'une quelconque des revendications 1 à 6, dans lequel l'orifice d'échantillon (112) est fixé à la première région d'extrémité (104) de la barrière imperméable aux fluides.

8. Ensemble de collecte de fluide selon l'une quelconque des revendications 1 à 7, dans lequel l'orifice d'échantillon (112) est un raccord à septum fendu.

9. Ensemble de collecte de fluide selon l'une quelconque des revendications 1 à 8, dans lequel l'orifice d'échantillon (112) fait partie d'un mécanisme luer-lock.

10. Ensemble de collecte de fluide selon l'une quelconque des revendications 1 à 9, dans lequel l'orifice d'échantillon (112) est au moins soit soudé par ultrasons, soit matricé à chaud sur la barrière imperméable aux fluides.

11. Ensemble de collecte de fluide selon l'une quelconque des revendications 1 à 10, dans lequel l'orifice d'échantillon (112) inclut un tube.

12. Ensemble de collecte de fluide selon l'une quelconque des revendications 1 à 11, dans lequel l'orifice d'échantillon est configuré pour être à l'état fermé lorsque l'orifice d'échantillon n'est pas fixé au collecteur de fluide et à l'état ouvert lorsque le collecteur de fluide est fixé à l'orifice d'échantillon.

13. Système de collecte de fluide, comprenant :
l'ensemble de collecte de fluide selon l'une quelconque des revendications 1 à 12 ; et
le collecteur de fluide (646).

14. Procédé d'utilisation du système de collecte de fluide selon la revendication 13, le procédé comprenant :
la fixation du collecteur de fluide à l'orifice d'échantillon ;
la réception de fluides corporels d'un individu à travers l'au moins une ouverture, dans la chambre et jusqu'au réservoir de fluide ;
l'élimination à travers l'orifice d'échantillon (112) dans le collecteur de fluide (144b) d'au moins une partie des fluides corporels qui sont présents dans le réservoir de fluide ;
la séparation du collecteur de fluide (144b) et de l'orifice d'échantillon (112) ;
le passage de l'orifice d'échantillon de l'état ouvert à l'état fermé ; et
l'élimination de sensiblement la totalité du reste des fluides corporels présents dans le réservoir de fluide à travers la sortie de fluide (118) en utilisant une force d'aspiration.
